# EUROPEAN PATENT APPLICATION

(11) **EP 2 653 867 A1**
(43) Date of publication of application: **23.10.2013**
(21) Application number: 12164315.9
(22) Date of filing: 16.04.2012
(51) Int. Cl.: G01N 33/543

(54) **Biotracer and its use in an immunoassay**

(71) Applicant: King Saud University, 11421 Riyadh (SA)
(72) Inventor: Mouffouk, Fouzi, 11421 Riyadh (SA); Abu-Salah, Khalid M., 11421 Riyadh (SA); Alrokayan, Salman A., 11421 Riyadh (SA)
(74) Representative: Goddar, Heinz J.

(57) **Abstract**

The present invention relates to a biotracer comprising polymeric micelles and its use in an immunoassay.

## Description

The present invention relates to a biotracer and its use in an immunoassay.

Immunoassays are widely used as analytical tools in clinical and pharmaceutical science. To be able to detect the interaction between two biomolecules, one of them has to be labeled. Various labelling agents have been applied of which radioisotopes are widespread, because of their inherent sensitivity. Other frequently used labelling agents are chemiluminescent compounds and enzymes (e.g. alkaline phosphatase or horseradish peroxidase) that convert an enzyme substrate into a measurable product.

Electrochemical detection is considered as one of the most reliable and efficient techniques to detect biomolecules within biological samples. However, its implementation in the field of immunoassay remains very limited compared to that of optical systems. Although several attempts were made in the past to develop immunosensors using enzymes converting a substrate into an electrochemical detectable product, so far no method is found that can rival with the existing systems. This failure is mostly attributed to the problems to achieve an acceptable signal-to-noise ratio due to: 1) the nonspecific adsorptions of the enzymes within the detection vicinity, 2) the nonspecific manner of whole antibody adsorption on the electrode surface during the immobilization step. Therefore, the reliability of this immunoassay test is dramatically affected.

To overcome the above problems, liposomes with marker molecules or enzymes encapsulated have been used, as disclosed in US 5,654,156. In these so-called liposome immunoassays, the liposome has to be conjugated to the antigen, either directly or indirectly. In the first case the liposome is covalently tethered to the antigen. In the latter, the liposome is covalently bound to an antibody or a secondary molecule, e.g. streptavidin or biotin.

Liposomes are small spherical structures that contain a polar surface and nonpolar interior, similar to the cell membrane. Conventional liposomes are characterized by a nonspecific reactivity with the milieu, whereas the sterically stabilized liposomes are relatively inert due to their surface coating with poly(ethylene) glycol (PEG) and therefore are less reactive or nonreactive to the environment. The major drawback of liposomes is their instability during use or during storage in biological media which can be reduced to their surface properties. Different strategies were investigated to increase the liposome stability. One of them consists in the modification of their external surface by optimisation of the lipid composition of the membrane, by incorporation of bioadhesive molecule (chitosan) in their membrane, by the polymerization of a two-dimensional network in the hydrophobic core of the membrane, by coating with nonporous silica or by the addition of surface active polymers. But so far, such improvements are very costly and add further complications to the fabrication process.

The second major problem of using conventional liposome in immunoassay is their leakage problem. Especially in case of low abundance protein detection, it was found that when small molecules are encapsulated inside liposome as tracers, these molecules have the tendency to leak into the outside medium. Such a problem will affect greatly the reliability of the performance of the immunoassay [Patrick Vermette et. al. Journal of Controlled Release 80 (2002) 179-195].

It is an object of the present invention to provide a biotracer for use in immunoassays which overcomes the drawbacks of the prior art. In detail, a more efficient, sensitive, cheap and reliable biotracer for use in immunoassay applications shall be provided.

This object is achieved by a biotracer comprising polymeric micelles, wherein a signal triggering and/or amplifying agent is encapsulated within the micelles and at least one functional group capable of reacting with an analyte is attached to the micelles.

In a preferred embodiment, the polymeric micelles have a hydrophobic inner part and a hydrophilic outer part.

Preferably, the biotracer is additionally comprising a solvent.

Preferably, the polymeric material of the micelles is selected from the group consisting of Poly(acrylic acid-b-acrylamide), Poly(acrylic acid-b-methyl methacrylate), Poly(n-butylacrylate-b-acrylic acid), Poly(sodium acrylate-b-methyl methacrylate), Poly(methacrylic acid-b-neopentyl methacrylate), Poly(methyl methacrylate-b-acrylic acid), Poly(methyl methacrylate-b-rnethacrylic acid), Poly(methyl methacrylate-b-N,N-dimethyl acrylamide), Poly(methyl methacrylate-b-sodium acrylate), Poly(methyl methacrylate-b-sodium methacrylate), Poly(neopentyl methacrylate-b-methacrylic acid), Poly(t-butyl methacrytate-b-ethylene oxide), Poly(butadiene(1,2 addition)-b-ethylene oxide), Poly(butadiene(1,2 addition)-b-(methylacrylic acid), Poly(butadiene(1,4 addition)-b-acrylic acid), Poly(butadiene(1,4 addition)-b-ethylene oxide, Poly(butadiene(1,4 addition)-b-sodium acrylate), Poly(butadiene(1,4 addition)-b-N-methyl 4-vinyl pyridinium iodide), Poly(isoprene-b-ethylene oxide) (1,2 and 3,4 -addition), Poly(isoprene-b-ethylene oxide) (1.4 addition), Poly(isoprene-b-N-methyl 2-vinyl pyridinium iodide), 4-Hydroxy benzoic ester Terminated Poly(butadiene(1,2 addition)-b-ethylene oxide), 4-Methoxy benzyolester Terminated Poly(butadiene(1,2 addition)-b-ethylene oxide) diblock copolymer, Poly(ethylene-b-ethylene oxide) (Hydrogenated Poly(1,4-butadiene)), Poly[(ethylene-eo-butene)-b-ethylene oxide] (Hydrogenated Poly(1,2-butadiene)), Poly[(propylene-co-ethylene-b-ethylene oxide)] 1,4-addition)} ethylene oxide), Poly(ethylene oxide-h-acrylic, acid), Poly(ethylene oxide-b-e-caprolactone), Poly(ethylene oxide-b-Lactide), Poly(ethylene oxide-b-methacrylic acid), Poly(ethylene oxide-b-methyl acrylate), Poly(ethylene axide-b-methyl methacrylate), Poly(ethylene oxide-b-N,N-dimethylaminoethylmethacrylate), Paly(ethylene oxide-b-propylene oxide), Poly(ethylene oxide-b-t-butyl acrylate), Poly(ethylene oxide-b-t-butyl methacrylate), Poly(ethylene oxide-b-tetrahydrofurfuryl methacrylate), Poly(ethylene oxide-b-2-ethyl oxazoline), Poly(ethylene oxide-b-2-hydroxyethyl methacrylate), Poly(ethylene oxide-b-2-methyl oxazoline), Paly[ethylene oxide-b-(methyl methacrylate-co-N,N-dimethylaminoethylmethacrylate)], Poly(ethylene oxide-b-4-vinyl pyridine) PEO end functional Methoxy, Poly(ethylene oxide-b-6-(4'-cyanobiphenyl-4-yloxy)hexyl methacrylate), Poly(ethylene oxide-b-11-[4-(4-butylphenylazo)phenoxy]-undecyl methacrylate), Poly(isobutylene-b-ethylene oxide), Poly(styrene-b-acrylamide), Poly(styrene-b-acrylic acid), Poly(styrene-b-cesium acrylate), Poly(styrene-b-cthylene oxide), Poly(styrene-b-ethylene oxide) Cleavable at the block unction , Poly(styrene-b-methaerylic acid), Poly(4-styrenesulfanic acid-b-ethylene oxide), Poly(styrene-b-N,N-dimethylacrylamide), Poly(styrene-b-N-isopropyl acrylamide), Poly(styrene-b-N-methyl 2-vinyl pyridinium iodide), Poly(styrene-b-N-methyl-4--vinyl pyridinium iodide), Poly(styrene-b-propylacrylic acid), Poly(styrene-b-sodium acrylate), Po-Iy(styrene-b-sodium methacrylate), Poly(p-chloromethyl styrene-b-acrylamide), Poly(styrene-co-p-chloromethyl styrene-b-acrylamide), Poly(styrene-co-p-chloromethyl styrene-b-acrylic acid), Poiy(N-isoprylacryIamide-b-ethylenc oxide), Poly(dimethylsiloxane-b-acrylic acid), Poly(dimethylsiloxane-b-ethylene oxide), Poly(dimethylsiloxane-b-methacrylic acid), Poly(2-vinyl naphthalene-b-acrylic acid), Poly(2-vinyl pyridine-b-ethylene oxide), Poly(2-vinyl pyridine-b-methyl acrylic acid), Poly(N-i-nethyl 2-vinyl pyridinium iodide-b-ethylene oxide), Poly(N-methyl 4-vinyl pyridinium iodide-b-znethyl methacrylate), Poly(4-vinyl pyridine-b-ethylene oxide) PEO end functional OH, Poly(vinyl pyrrolidone-b-D/L-lactide) or a mixture thereof, most preferably Poly(ethylene oxide-b- ε -caprolactone).

Even preferred, the signal triggering and/or amplifying agent is a redox-activ compound and/or a fluorescent dye, more preferably a redox-active compound, most preferably ferrocene or hydrochinone.

In a further preferred embodiment, the solvent is water or a mixture of water and an organic solvent, most preferably the solvent is water.

Further preferred, the functional group is selected from a prosthetic group, a group of an antibody, an antigen, a peptide, a protein, DNA, an amino acid, a sugar, a oligo- or polysaccharide, a nucleic acid, a co-factor and/or a vitamin.

Even preferred, the analyte is a biomolecule, preferably selected from a molecule having a prosthetic group, an antibody, an antigen, a peptide, a protein, a DNA, an amino acid, a sugar, a oligo- or polysaccharide, a nucleic acid, a co-factor and/or a vitamin.

The object is further achieved by an immunoassay comprising the steps:
a) providing beads having binding groups capable of binding to an analyte,
b) mixing the beads with an analyte containing solution,
c) mixing of the solution obtained in step b) with the biotracer according to any of the claims 1 to 8,
d) separating bead-analyte-biotracer-complexes from the solution obtained in step c),
e) releasing the signal triggering and/or amplifying agent, and
f) detecting the signal triggering and/or amplifying agent.

In a preferred embodiment, the beads are magnetic beads.

Even preferred, the binding is attached to the bead surface by an anchor group and the connection between the binding group and the anchor group is optionally made by a ligand.

Further preferred, the binding group is a biomolecule, preferably the binding group is a prosthetic group, an antibody, an antigen, a peptide, a protein, DNA, an amino acid, a sugar, a oligo- or polysaccharide, a nucleic acid, a co-factor and/or a vitamin.

Even preferred, separating is magnetic separating.

Finally preferred, the signal triggering and/or amplifying agent is released from the micelle by ultrasonic treatment, change in pH, change in solvent composition, light irradiation and/or addition of a detergent.

It was surprisingly found that the inventive biotracer solves the problem by providing an efficient, cheap and reliable way to trigger and/or amplify signals in immunoassay applications, in particular by providing a significant increased sensitivity compared to the prior art. Further characteristics and advantages of the invention result from the detailed description of preferred embodiments, particularly in context with the examples and the attached drawings, wherein
Fig. 1 shows a schematic illustration of an immunoassay according to the invention.
Fig. 2 shows a Transmission Electron Micrograph (TEM) of a biotracer formed by PE045-b-PCL23 micelles not encapsulating any signal triggering and/or amplifying agent and not containing functional groups, wherein the bar scales 50 nm.
Fig. 3 shows a histogram displaying the size distribution of a biotracer of PE045-b-PCL23 micelles not encapsulating any signal triggering and/or amplifying agent and not containing functional groups, wherein the particle sizes were measured by Dynamic Lights Scattering (DLS).
Fig. 4 shows a histogram displaying the size distribution of a biotracer of PE045-b-PC23 micelles encapsulating ferrocene as signal triggering and/or amplifying agent but not containing functional groups, wherein the particle sizes were measured by Dynamic Light Scattering (DLS).

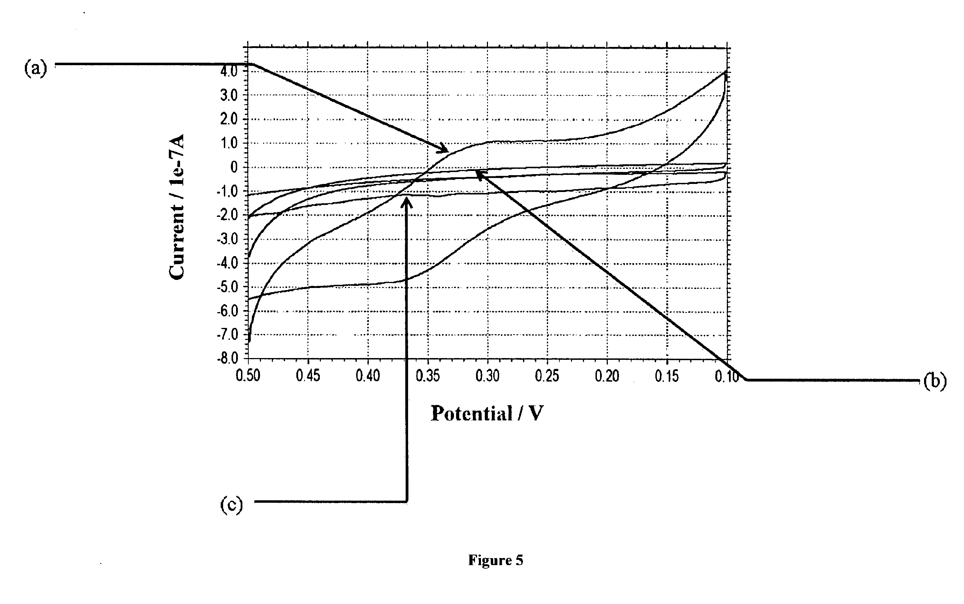

Fig. 5 shows different cyclic voltammograms, wherein (a) is a cyclic voltammogram of biotin bioconjugated polymeric micelles after the release of ferrocene molecules, (b) is a cyclic voltammogram of polymer micelles encapsulating ferrocene molecules but not containing functional groups in the presence of a bead-analyte-eaznplex, wherein the analyte is avidin and (c) is a cyclic voltammogram of a bead-analyte-complex to which a ferrocene group is attached by a spacer having a terminal biotin head group and which can interact with the avidin molecules of the bead-analyte-complex (as a control experiment to demonstrate the ability of the new bioassay to amplify the binding event between biotin and avidin).
Fig. 6 shows the fluorescence intensity of a PE045-b-PCL23 biotracer encapsulating a fluorescent dye in dependence of the pH of the applied solution.
Fig. 7 shows the fluorescence intensity of a PE045-b-PCL23 biotracer encapsulating a fluorescent dye in dependence of the temperature.

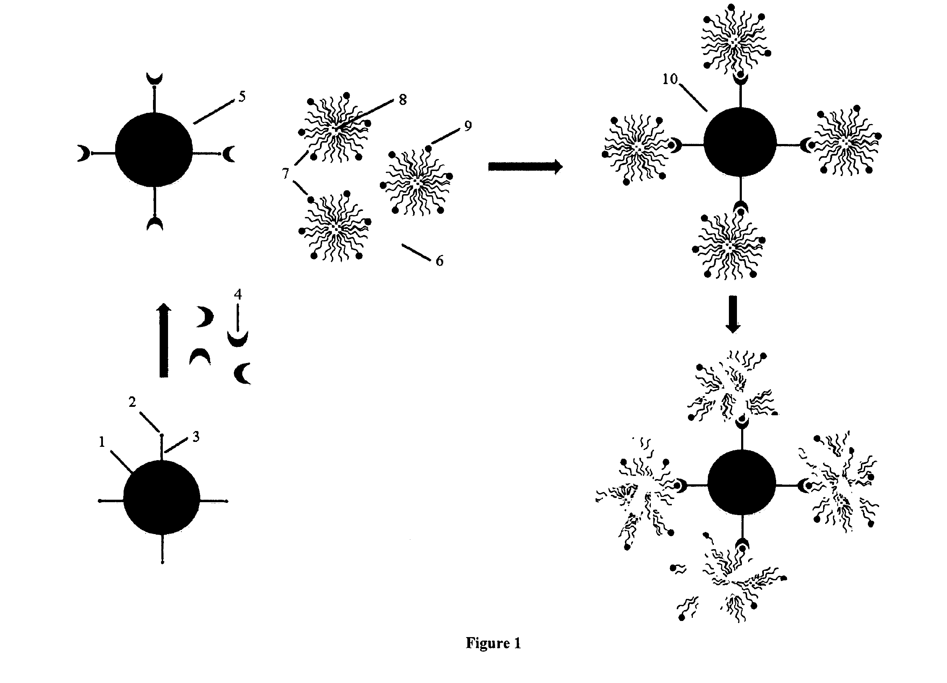

Reference is now made to Fig. 1 which illustrates an immunoassay according to the invention.

In step a) of the inventive immunoassay, beads 1 having binding groups 2 are provided. The beads 1 are preferably coated by ligand 3 molecules, wherein at least parts of the ligands 3 have to have one or more binding groups 2 capable of binding to appropriate analyte 4 molecules surrounding the ligand-coated beads 1 in a solution.

The beads 1 have sizes from a few nanometers to several micrometers and consist of a material allowing an easy separation of the beads 1 from a surrounding environment. For example, using magnetic beads allows easy separation by the application of an external magnetic field and subsequent decantation. Possible magnetic materials are for example iron oxides. Many methods are known in the prior art to prepare beads in a nano- or micro meter scale, for example mechanical grinding of an appropriate material, sol-gel synthesis using chemical precursors, hydrothermal synthesis, micro emulsion technique etc.

The beads 1 are preferably coated by ligand molecules 3 having a binding group 2. Coating of the beads 1 can preferably be achieved by binding of the ligand 3 via an appropriate anchor group to the beads 2 surfaces. For example, it is known that amine groups feature a high affinity for binding to metal oxide surfaces. As well, it is known that noble metal surfaces (e.g. gold or palladium) can be easily coated by molecules containing thiol groups. Coating of the metal beads 1 can for example be carried out by exposing the bare beads 1 to a solution containing ligands having an appropriate anchor group, by the addition of ligands 3 during preparation of the beads 1 from chemical precursors, by ligand exchange etc.

At least parts of the ligand 3 molecules have to have binding groups 2 which are able to bind to the analyte 4 surrounding the beads 1. In principle, every organic or biomolecule allowing a specific interaction with another substance and which can be bound to the ligands 3 can be used as binding group 2 in the present invention. The binding group 2 has to be located in the ligand molecule 3 in a way to facilitate interaction with the environment. Due to this, binding groups 2 which are terminal bound to the ligand 3 are preferred. The binding groups 2 can be attached to the ligand 3 molecule by suitable organic reactions, for example substitutions, additions etc. Likewise, the binding groups can additionally comprise a specific rest group enabling binding to the ligand 3 molecule via coupling reactions, for example by the formation of peptide bounds, "click" reaction, Meek-coupling etc.

Binding groups 2 able to bind directly to the beads surfaces as well as to the analyte, can of course, be attached to the beads without using ligands 3.

In step b) of the reaction, the provided beads 1 having a binding group 2 are mixed with a solution containing an analyte 4. In a typical embodiment, the analyte 4 containing solution will be an aqueous solution which can in addition to the analyte 4 also contain other organic and/or biomolecules, buffer systems etc.

The analyte 4 is bound to the beads 1 via the binding groups 2. The nature of the analyte, 4 has to be considered by choosing the binding group 2 when providing beads 1. Numberless examples for appropriate binding group-analyte pairs are known in the art, particularly in biochemistry under the heading of "lcey-loclc principle". For example, appropriate antigen-antibody or cofactor-protein couples can advantageously be used as binding group 2 and analyte 4.

Binding of the analyte 4 to the beads 1 via the binding group 2 can be achieved by any kind of interaction allowing sufficient strong binding, for example by covalent binding, ionic interaction, appropriate weak interaction etc.

In the next step of the method, the solution obtained in step b) containing the bead-analyte-complex 5 is mixed with biotracers 6 of the invention. The biotracer 6 comprises polymer micelles 7, a signal triggering and/or amplifying agent 8 and optionally a solvent.

Each micelle 7 is formed by several polymer molecules which can be of a different type. The signal triggering and/or amplifying agent 8 is encapsulated in the micelles 7. Appropriate polymers and signal triggering and/or amplifying agents 8 are listed above.

The micelles 7 are preferably surrounded by a stabilizing solvent. Since a preferred purpose of the inventive method is the use in biochemistry, the preferred solvent system will be water or an aqueous solution, for example a buffering solution.

The amount of signal triggering and/or amplifying agent 8 encapsulated in the micelles 7 has to be sufficient to allow an efficient detection in the last step of the method. The required amount of signal amplifying agent 8 depends on its chemical nature and on the chosen method of detection.

At least parts of the micelle 7 forming polymer molecules have to have a functional group 9 which allows binding to the bead-analyte-complex 5. As in case of the binding group 2, every organic or bimolecule allowing a specific interaction with the analyte 4 is conceivable as a possible functional group 9, as long as a specific interaction according to the "key-lock principle" is provided. Binding of the micelles 7 to the bead-analyte-complex 5 via binding of the functional group 9 to the analyte 4 leads to the formation of a bead-analyte-biotracer-complex 10.

The bead-analyte-biotracer-complex 10 is separated from the remaining solution in an appropriate way. For example, if magnetic beads 1 are used, separation can be carried out by applying an magnetic field to the solution, for example by using a rod magnet, and subsequent decantation.

The signal triggering and/or amplifying agent is released from the micelles by treatment of the complex 10 in a way allowing the destruction of the micelles 7. Appropriate methods for destroying the micelles 7 are listed above.

In the last step of the method, the released signal triggering and/or amplifying agent 8 will be detected in an appropriate way. The detection method will depend on the chemical and physical nature of the signal trigerring and/or amplifying agent 8. For example, if the signal triggering and/or amplifying agent 8 is a redox-active substance, an electroanalytic detection method, for example cyclic voltammetry, will be the detection method of choice. In case that the signal triggering and/or amplifying agent 8 is a fluorescent dye, it will be detected by fluorescence spectroscopy.

The signal triggering and/or amplifying agent triggers a signal in that it is detectable by a specific method and amplifies the signal in that the number of signal triggering and/or amplifying agent molecules exceeds the number of analyte molecules.

It is expected that the immunoassay's sensitivity can be improved through alternative embodiments. In one embodiment, alternative hydrophobic dyes with enhanced electrochemical features can be selected from the list of electrochemical active molecules presently available. Its anticipated that specific electroactive molecules can be matched with specific antibodies to improve the sensitivity to the analyte and to test multiple analytes in a single sample (by reading different redox/oxidation potential).

### Experiments

To demonstrate the proof of the inventive concept, ferrocene loaded polymeric micelles were prepared from block co-polymer of poly(caprolactone)₂₂-poly(ethylene oxide)₄₅ and the corre-spending bioassay was developed to detect avidin in serum-like solution (avidin plus BSA in PBS buffer). In brief, the assay involves the incubation of biotinylated magnetic beads with target molecules from a pre-prepared solution of serum mimic (avidin and BSA). After washing off non-specifically bound biomolecules, biotin labeled micelles are incubated with the beads, followed by washing off unbound micelles. The presence of target molecules was detected by 1) adding organic solvent (THF) or detergent to the solution and 2) measuring the electrochemical signal of the released ferrocene molecules in solution. Using such an assay, we were able to detect avidin up to femtomolar levels. Considering the molecular weight of avidin, which is ∼ 66-69 kD, the assay can reliably detect 1,4 x 10⁻¹⁵ M af avidin without any optimization.

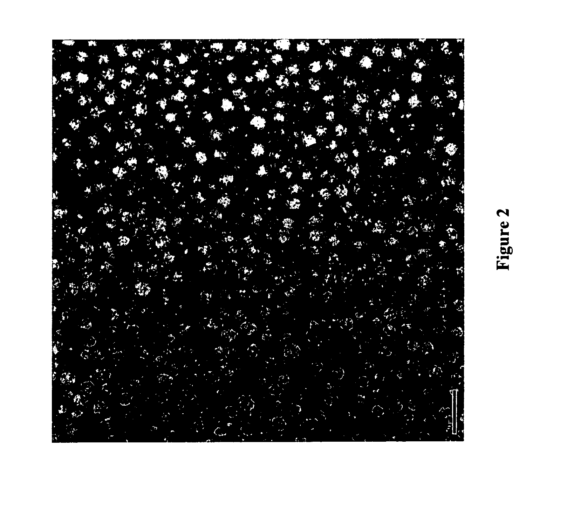

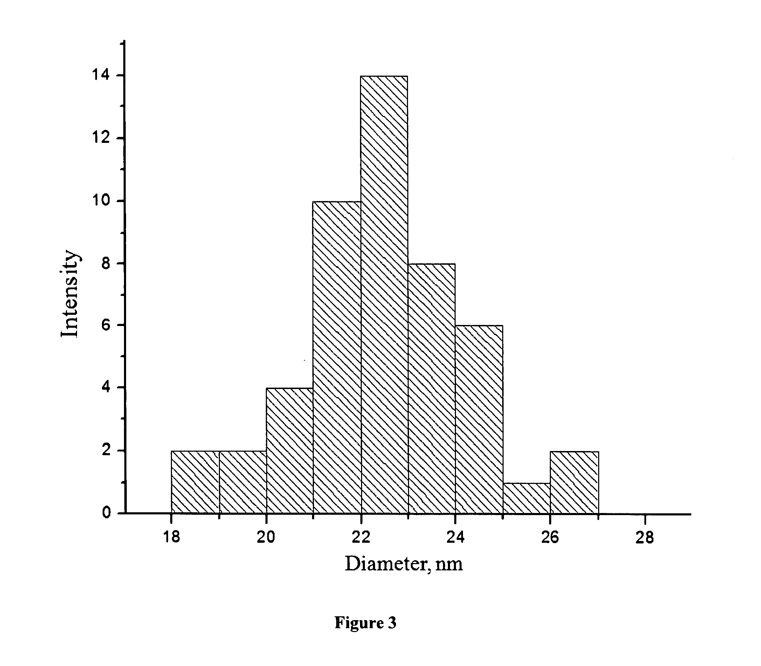

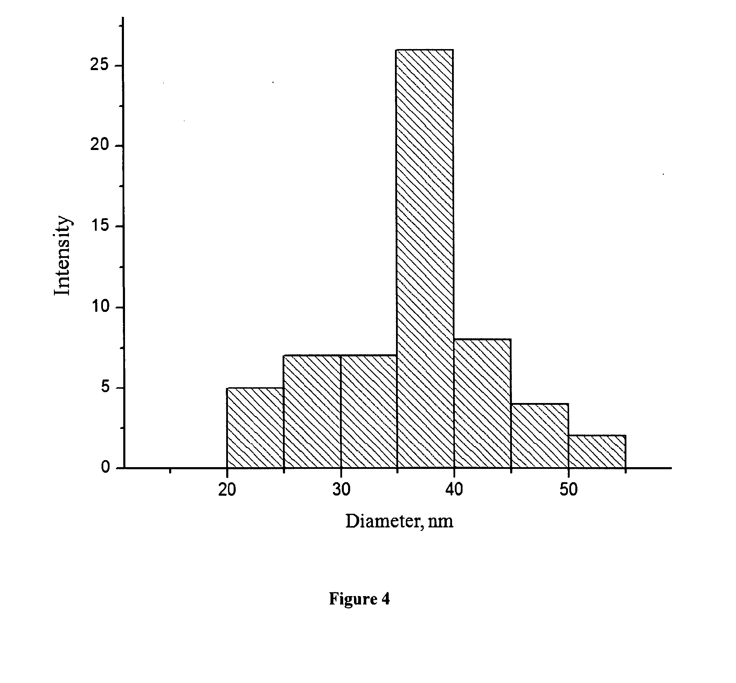

The preparation of PCL23-b-PEO4S micelles and electroactive dye encapsulation was carried out using a modified procedure as mentioned below. In brief, the solution of PCL23-b-PE045 in DMF was prepared and the micelle formation was achieved by adding deionized water at a rate of 10 µl/30s at room temperature. The polymeric micelles were purified by dialyzing against water. Polymeric micelles with electroactive dye encapsulated were prepared under the same conditions, except that the micelles were formed in the presence of 0.5 mg of ferrocene. The native polymeric micelles have an average diameter of 24 nm (Fig. 3), while the dye encapsulating polymeric micelles exhibit significantly increased diameters with an average value of 40 nm (Fig. 4). Ferrocene encapsulated polymeric micelles are also visualized under Transmission Electron Microscope (TEM) by negatively staining with 1% uranyl acetate (Fig. 2). The size of the polymeric micelles obtained from TEM matches the values derived from the DLS studies. The electroactive dye encapsulation and proof of no leakage was confirmed by toxicity studies of the dye encapsulated micelles with embryonic stem cells. It is known that ferrocene molecules are very toxic to stem cells. When present in the media, 50 µM of ferrocene induced overall cell death in three days. However, upon the encapsulation of ferrocene molecules within polymeric micelles (0.5 mM), the polymeric micelles minimized the dye's toxicity against embryonic stem cells and cell colony growth remains normal for 3 days. The results suggest that the dye molecules are encapsulated and retained inside the micelles.

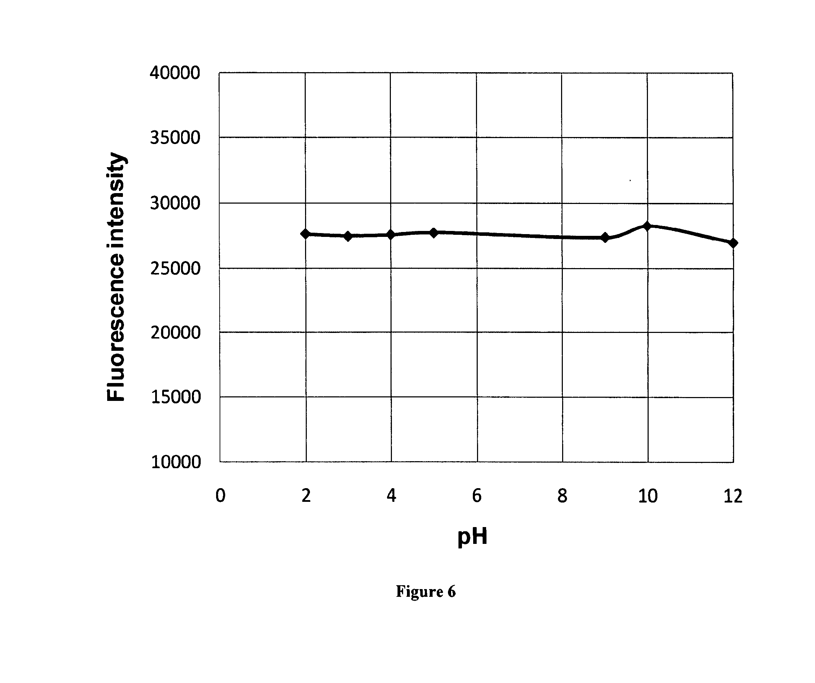

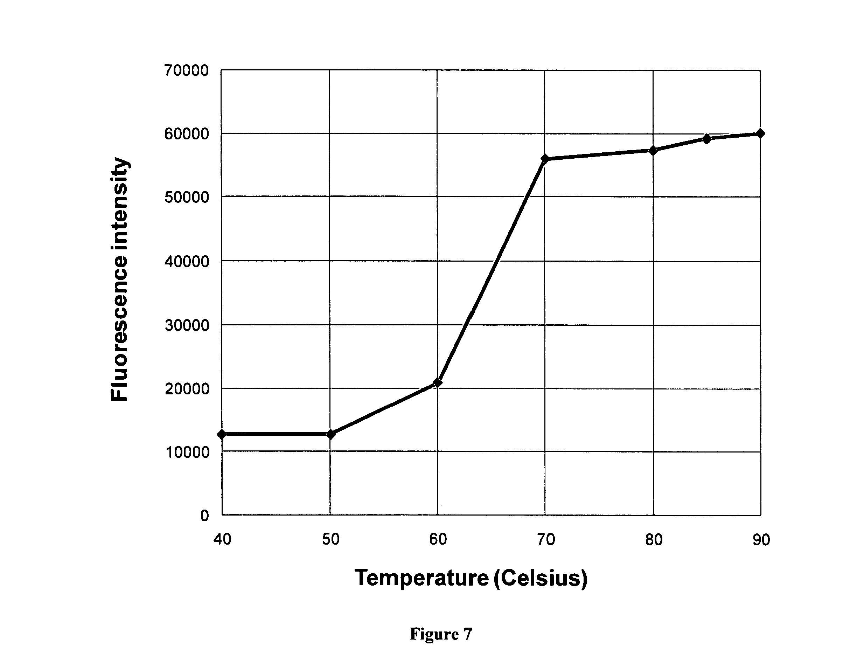

To test the release mechanism, the electroactive dye was replaced by a fluorescent dye. The polymeric micelles were loaded with dimethyl amino stilbene acetyl ketone dye (DSAK). The fluorescence of DSAK is self-quenched when the dye molecules are encapsulated within the micelles, whereas the fluorescence signal is enhanced dramatically when the molecules are released to the solution. To use this signal amplification process for a novel bioassay, several approaches for releasing the dye, including pH variation, temperature changes, and exposure of the micelles to organic solvents or ultrasound were probed. It was found that dye-encapsulating micelles remained stable against pH variation (from pH 2 to 12) and temperature changes from 20 to 65°C (Fig. 6, 7). No fluorescence increase (i.e., dye leakage) was observed under the above conditions. However, when the dye-encapsulated micelles were treated with organic solvents, such as THF or detergent, the fluorescence intensity of the bulk solution increased dramatically, indicating substantial release of dye molecules into the solution. Similar observations were obtained when the micelle solution was exposed to probe sonification at a frequency of 20 KHz at 0°C for 5 minutes. However, it is possible to render these polymeric nanocontainers sensitive to light or to pH change and to use an alternative procedure to release the electroactive dye from the container, simply by using other type of polymeric micelles that are responsive to pH change or to light. Based on the average molecular mass difference Δm (4.8E⁻¹⁷grams) between the micelle with the ferrocene (m₂) and the micelle without (m₁) that has derived from Static light scattering and the molecular weight of the one molecule of dye (4,4E⁻²² grams), we estimated the number of dyes encapsulated inside one micelle with a diameter of 37 nm to be ∼110,000 molecules. The release of these dye molecules to the solution provides a significant signal amplification process for monitoring the antigen-antibody binding event, which can be harnessed to develop a novel, sensitive bioassay.

Electrochemical experiments were carried out in a two-compartment cell in which the reference electrode (RE) compartment was connected to the main cell by a ceramic frit. A saturated calomel reference electrode was employed. The liquid junction potential formed is assumed to be small and constant throughout. The working electrodes (WE) used were platinum disc electrodes (10 µm diameter) and rinsed several times with distilled water and allowed to dry under argon. These electrodes were rinsed with the dry electrolyte before placing them in the electrochemical cell. The built-in Luggin capillary allowed the placement of the RE as close as possible to the WE in order to minimize the uncompensated solution resistance. The counter electrode (CE) used was platinum gauze. Cyclic voltammetry (CV) studies were carried out in this two-compartment, three-electrode configuration using an Autolab PG-30 potentiostat with GPE and FRA software programmes (Eco Chimie).

### Example 1) Preparation of polymeric micelles.

20 mg of PEO₄₅-*b*-PCL₂₃ block copolymers was dissolved in 0.38 g of DMF and stirred for 3 h. Deionized water was added at a rate of 10 µl / 30s for a total of 1.6 ml to induce micellization. The aqueous solution was dialyzed (10K MWCO, *Pierce Biotech*.) against deionized water for 2 days. After dialysis, the polymer solution was diluted to 0.67% (w/w) by water for subsequent experiments.

### Example 2) Preparation of bioconjugated micelles,

### a)- Bioconjugation of Polyethylene glycol with biotin (biotin-PEO).

Methoxypolyethylene glycol succinate N-hydroxysuccinimide ester (0.5 mg) were added to the solution of biotin-poly(ethyleneglycol) amine (100 µg in 0.5 ml of DMF). The mixture was then gently stirred overnight in slow tilt rotation at 4°C.

### b)- Preparation of loaded bioconjugated micelles.

20 mg of PEO₄₅-b-PCL₂₃ block copolymers, and 0.5 mg of ferrocene were dissolved in 0.5 ml of the freshly prepared biotin-PEO. The mixture was stirred for 3 h at room temperature. 2 ml a aqueous solution were added at a rate of IO µl / 10s to induce micellization. The yielded solution was then placed in a Dialysis cassette of 1OK MWCO and dialyzed against deionized water for 2 days at 4°C. After dialysis, the solution was diluted 5 times in PBS buffer.

### Example 3) Avidin detection.

100 µg of the bzoti3z-poly(ethyleneglycol) amine was added to tosylactivated magnetic beads (8x10⁷ beads, 400 µl) in 0.1 M borate buffer (pH 9.5). The mixture was then incubated for 16-24 hours with slow tilt rotation at 4°C. Unbound biotin molecules were removed using a magnetic separator. The beads were then washed twice with PBS (phosphate buffered saline, pH 7.4) containing 0.1% (w/v) BSA for 5 minutes each at 4°C, followed by blocking the remaining active tosyl- groups with 0.2 M Tris buffer (pH 8.5) containing 0.1% (w/v) BSA. The beads were finally washed with PBS (pH 7.4).

The freshly prepared biotin-coated beads were incubated with mimic serum solution (avidin 0.1 units/ml and BSA 10units/ml) for 2 hours. The unbound avidin was then removed by washing four times in PBS followed by magnetic separation. Subsequently, 200 µl of ferrocene loaded polymeric micelles and conjugated with biotin was added to the beads. The mixture was gently stirred for 2 hours at 4°C. The unbound bioconjugated micelles were removed by magnetic separation. After washing the beads four times in PBS, the ferrocene molecules were released from the polymeric micelles by the addition ofTHF (1 ml). Redox signal of ferrocene molecules was recorded and measured inside electrochemical cell using Autolab potentiostat.

Also series of control experiments were conducted: 1) virtually no electrochemical signals were observed when biotinylated magnetic beads were incubated with bioconjugated micelle samples, indicating that there are no nonspecific interactions between the polymeric micelles and the magnetic beads. 2) When the avidin antigen was replaced with BSA (1mg/ml), again we did not observe any significant electrochemical signals. 3) When biotinylated polymeric micelles were replaced with plain polymeric micelles in the test, we did not observe any significant electrochemical signals. 4) Also when biotin conjugated micelles were replaced with biotin conjugated with single ferrocene molecule, we observed a sharp drop in the electrochemical signal, demonstrating the amplification affect that these polymeric micelles.

The studies showed that the polymeric micelles are stable against leakage and have a phase transition temperature around 60°C. The polymeric micelles can encapsulate around 10⁵ to 10⁶ dye molecules and the dye molecules can be released by ultrasound, organic solvents, pH change or by light exposure (it depend on the chemical structure of the copolymer). A bio-detection assay was developed based on the polymeric micelles with femtomolar sensitivity and capable of detecting avidin from mimic serum. Although this new bioassay can be used for the detection of almost any antigen of interest that found is found in biological fluid such as plasma lysates, saliva, urine, mucous or the like.

Features of the present invention disclosed in the description and/or the claims may both separately and in any combination thereof be material for realizing the invention in various forms thereof.

## Claims

1. Biotracer comprising polymeric micelles, wherein a signal triggering and/or amplifying agent is encapsulated within the micelles and at least one functional group capable of reacting with an analyte is attached to the micelles.

2. Biotracer according to claim 1, wherein the polymeric micelles have a hydrophobic inner part and a hydrophilic outer part.

3. Biotracer according to claim 1 or 2, additionally comprising a solvent.

4. Biotracer according to any of the preceding claims, wherein the polymeric material of the micelles is selected from the group consisting of poly(acrylic acid-b-acrylamide), poly(acrylic acid-b-methyl methacrylate), poly(n-butylacrylate-b-acrylic acid), poly(sodium acrylate-b-methyl metbacrylate), poly(methacrylic acid-b-neopentyl methacrylate), poly(methyl methacryiate-b-acrylic acid), poly(methyl methacrylatc-b-methacrylic acid), poly(methyl methacrylate-b-N,N-diznethyl acrylamide), poly(methyl methacrylate-b-sodium acrylate), poly(methyl methacrylate-b-sodium methacrylate), poly(neopentyl mcthacrylate-b-methacrylic acid), poly(t-butyl mcthacrylate-b-ethylene oxide), poly(butadiene(1,2 additian)-b-ethylene oxide), poly(butadiene(1,2 addition)-b-(methylacrylic acid), poly(butadiene(1,4 addition)-b-acrylic acid), poly(butadiene(1,4 addition)-b-ethylcne oxide, poly(butadiene(1,4 addition)-b-sodium acrylate), poly(butadiene(1,4 addition)-b-N-methyl 4-vinyl pyridinium iodide), poly(isoprene-b-ethylene oxide) (1,2 and 3,4 -addition), poly(isopz°eaae-b-ethylene oxide) (1.4 addition), poly(zsoprene-b-N-methyl 2-vinyl pyridinium iodide), 4-Hydroxy benzoin ester terminated poly(butadiene(1,2 addition)-b-ethylene oxide), 4-Methoxy benzyolester Terminated poly(butadiene(1,2 addition)-b-ethylene oxide) diblock copolymer, poly(ethylenc-b-ethylenc oxide) (hydrogenated poly(1,4-butadiene))., poly[(ethylene-co-butene)-b-ethylene oxide] (hydrogenated poly(1,2-butadieiie)), poly[(propylene-co-ethylene-b-cthylene oxide)] 1,4-addition) ethylene oxide), poly(ethylene oxide-b-acrylic acid), poly(ethylene oxide-b-ε-caprolactone), poly(ethylene oxide-b-Lactide), poly(ethylene oxide-b-methacrylic acid), poly(ethylcne oxide-b-methyl acrylate), poly(ethylene oxide-b-methyl methacrylate), poly(ethylene oxide-b-N,N-dimethylarninoethylmethacrylate), poly(ethylene oxide-b-propylene oxide), poly(ethylene oxide-b-t-butyl acrylate), poly(ethylene oxide-b-t-butyl methacrylate), laoly(ethylene oxide-b-tetral-Lydrofurturyl methacrylate), poly(ethylene oxide-b-2-ethyl oxazoline), poly(ethylene oxide-b-2-hydroxyethyl methacrylate), poly(ethylene oxide-b-2-methyl oxazoline), poly[ethylene oxide-b-(methyl methacrylate-co-N,N-dimethylaminoethylmethacrylate)], poly(ethylene oxide-b-4-vioyl pyridine) PEO end functional Methoxy , poly(ethylene oxide-b-6-(4'-cyanobiplzenyl-4-yloxy)hexyl methacrylate), poly(ethylene oxide-b-11-[4-(4-butylphenylazo)phenoxy]-undecyl methacrylate), poly(isobutylene-b-ethylene oxide), poly(styrene-b-acrylarnidc), poly(styrene-b-acrylie acid), poly(styrene-b-cesium acrylate), poly(styrene-b-ethylene oxide), poly(styrcne-b-ethylene oxide) Cleavable at the block junction, poly(styrene-b-methacrylic acid), poly(4-styrenesulfonic acid-b-ethylene oxide), poly(styrene-b-N,N'dimethylacrylamide), poly(styrene-b-N-isopropyl acrylamide), poly(styrene-b-N-rnethyl 2-vinyl pyridinium iodide), poly(styrene-b-N-methyl-4-vinyl pyridinium iodide), poly(styrene-b-propylacrylic acid), poly(styrene-b-sodium acrylate), poly(styrene-b-sodiul11 methacrylate), poly(p-chloromethyl styrene-b-acrylamide), poly(styrene-co-p-chloromethyl styrene-b-acrylamide), poly(styrene-co-p-chloromethyl styrene-b-acrylic acid), poly(N-isoprylacrylamide-b-ethylene oxide), poly(dimethylsiloxane-b-acrylic acid), poly(dimethylsiloxane-b-ethylene oxide), poty(dimethylsiloxanc-b-methacrylic acid), poly(2-vinyl naphtalene-b-acrylic acid), poly(2-vmyl pyridine-b-ethylene oxide), poly(2-vinyl pyridiiie-b-methyl acrylic acid), poly(N-methyl 2-vinyl pyridinium iodide-b-etllylene oxide), poly(N-methyl 4-vinyl pyridinium iodide-b-methyl methacrylate), poly(4-vinyl pyridine-b-ethylene oxide) PEO end functional OH, poly(vinyl pyrrolidone-b-D/L-lactide) or mixtures thereof, most preferably poly(ethylene oxide-e-y-caprolactone).

5. Biotracer according to any of the preceding claims, wherein the signal triggering and/or amplifying agent is a redox-activ compound and/or a fluorescent dye, preferably a redox-active compound, more preferably ferrocene or hydrochinone.

6. Biotracer according to any of the preceding claims, wherein the solvent is water or a mixture of water and an organic solvent, most preferably the solvent is water.

7. Biotracer according to any of the preceding claims, wherein the functional group is selected from a prosthetic group, a group of an antibody, an antigen, a peptide, a protein, DNA, an amino acid, a sugar, a oligo- or polysaccharide, a nucleic acid, a co-factor and/or a vitamin.

8. Biotracer according to any of the preceding claims, wherein the analyte is a biomolecule, preferably selected from a molecule having a prosthetic group, an antibody, an antigen, a peptide, a protein, a DNA, an amino acid, a sugar, a oligo- or polysaccharide, a nucleic acid, a co-factor and/or a vitamin.

9. Immunoassay comprising the steps:
a) providing beads having binding groups capable of binding to an analyte,
b) mixing the beads with an analyte containing solution,
c) mixing of the solution obtained in step b) with the biotracer according to any of the claims 1 to 8,
d) separating bead-analyte-biotracer-complexes from the solution obtained in step C).
e) releasing the signal triggering and/or amplifying agent, and
f) detecting the signal triggering and/or amplifying agent.

10. Method according to claim 9, wherein the beads are magnetic beads.

11. Method according to claim 9 or 10, wherein the binding group is attached to the bead by an anchor group and the connection between the binding group and the anchor group is optionally made by a legand.

12. Method according to any of the claims 9 to 11, wherein the binding group is a biomolecule, preferably the binding group is a prosthetic group, an antibody, an antigen, a peptide, a protein, DNA, an amino acid, a sugar, a oligo- or polysaccharide, a nucleic acid, a co-factor and/or a vitamin.

13. Method according to claim 9, wherein separating is magnetic separating.

14. Method according to any of the preceding claims 9 to 13, wherein the signal triggering and/or amplifying agent is released from the micelle by ultrasonic treatment, change in pH, change in solvent composition, light irradiation and/or addition of a detergent.
